# EUROPEAN PATENT APPLICATION

(11) **EP 4 819 149 A1**
(43) Date of publication of application: **07.10.2026**
(21) Application number: 26169973.0
(22) Date of filing: 31.03.2026
(51) Int. Cl.: G16H 10/20, G06N 3/10, G06N 20/00, G16H 10/60, G16H 15/00, G16H 50/70

(54) **DEVELOPING SOFTWARE FOR CALIBRATING A NEURAL NETWORK TO OUTPUT NEAR REAL-TIME PREDICTIONS OF CLINICAL TRIAL STUDY ENROLLMENT**

(30) Priority: 02.04.2025 US 202519098528
(71) Applicant: Medidata Solutions, Inc., New York, NY 10014 (US)
(72) Inventor: LIU, Jingshu, New York, NY, 10014 (US); REN, Qingyun, New York, NY, 10014 (US); BUKA, Robert, New York, NY, 10014 (US); KANN, Sarah, New York, NY, 10014 (US); KATZ, Laura, New York, NY, 10014 (US); LEI, Amy, New York, NY, 10014 (US)
(74) Representative: Fish & Richardson P.C.

(57) **Abstract**

An apparatus for facilitating development of software to use a neural network in generating output indicative of a near real-time prediction of clinical study enrollment, comprises one or more processors; and one or more hardware storage devices storing instructions that are executable by the one or more processors to perform one or more first operations comprising: receiving, in a development environment, one or more instructions that are executable by the one or more processors to perform one or more second operations comprising: receiving, from the one or more user interfaces, input data corresponding to data input into the one or more portions of the user interface; inputting, into a neural network, the target schema, received input data specifying the given clinical trial, the enrollment data and the historical data; and receiving, from the neural network, prediction data specifying one or more near real-time predictions of enrollment for the clinical trial.

## Description

### TECHNICAL FIELD

This description generally relates to systems and methods for developing software for calibrating a neural network.

### SUMMARY

Implementations according to this disclosure include an apparatus for facilitating development of software to use a neural network in generating output indicative of a near real-time prediction of clinical study enrollment, including: one or more processors; and one or more hardware storage devices storing instructions that are executable by the one or more processors to perform one or more first operations including: receiving, in a development environment, one or more instructions that are executable by the one or more processors to perform one or more second operations including: causing rendering of one or more user interfaces with one or more input portions for input of data specifying a clinical trial; receiving, from the one or more user interfaces, input data corresponding to data input into the one or more portions of the user interface, with the input data received specifying a given clinical trial; receiving, from the one or more user interfaces, a target schema for the given clinical trial; accessing, from one or more hardware storage devices, historical data and enrollment data for the given clinical trial, with the historical data being from one or more studies conducted in a disease area associated with the clinical trial, and with the enrollment data specifying actual enrollment in the clinical trial; inputting, into a neural network, the target schema, received input data specifying the given clinical trial, the enrollment data and the historical data; receiving, from the neural network, prediction data specifying one or more near real-time predictions of enrollment for the clinical trial; and outputting to one or more user interfaces one or more visualizations of the received prediction data.

In some implementations, the clinical trial is associated with one or more sites, wherein the accessed enrollment data is for a given one of the one or more sites, and wherein the prediction data specifies one or more near real-time predictions of enrollment for the clinical trial for the given one of the one or more sites. The target schema specifies an enrollment target, an activation date and/or a completion date. The neural network is a recurrent neural network. The one or more visualizations provide a first view at a study level or a second view at a country level. The study level may include a clinical trial study level.

In other implementations, a method implemented by a data processing system for generating a target schema for use with a neural network to generate output indicative of a near real-time prediction of clinical study enrollment, includes causing rendering of one or more user interfaces with one or more input portions for input of data specifying a clinical trial; receiving, from the one or more user interfaces, input data corresponding to data input into the one or more portions of the user interface, with the input data received specifying a given clinical trial; based on the received input data, generating, by a data processing system, a target schema for the given clinical trial, with the target schema specifying a target enrollment and a completion date for the given clinical trial; accessing, from one or more hardware storage devices, historical data and enrollment data for the given clinical trial, with the historical data being from one or more studies conducted in a disease area associated with the clinical trial, and with the enrollment data specifying actual enrollment in the clinical trial; inputting, into a neural network, the target schema, the enrollment data and the historical data; receiving, from the neural network, prediction data specifying one or more near real-time predictions of enrollment for the clinical trial; and outputting to one or more user interfaces one or more visualizations of the received prediction data.

In these implementations, the clinical trial is associated with one or more sites, wherein the accessed enrollment data is for a given one of the one or more sites, and wherein the prediction data specifies one or more near real-time predictions of enrollment for the clinical trial for the given one of the one or more sites. The target schema specifies an enrollment target, an activation date and/or a completion date. The actions include calibrating the neural network by: accessing, from a hardware storage device, clinical trial data; segmenting the clinical trial data into test data and training data; for the training data, identifying one or more features of the training data; encoding the one or more features; transmitting at least a portion of the one or more encoded features to one or more hidden layers of the neural network; based on an output of the one or more hidden layers, determining one or more values of one or more hyperparameters of the neural network; and repeating the transmitting and the determining until the neural network outputs one or more values satisfying one or more thresholds.

In these implementations, the actions include training the neural network to predict the one or more near real-time predictions using a supervised learning technique based on training data, wherein the neural network is configured to receive as input the target schema, input data specifying the given clinical trial, the enrollment data and the historical data and to process the input to generate an output that specifies one or more near real-time predictions of enrollment for the given clinical trial, wherein the neural network includes a plurality of artificial neurons that are connected through edges and are aggregated into a plurality of neural network layers including at least an input layer and an output layer, wherein each of the edges is configured to transmit a signal from one artificial neuron to another artificial neuron, and wherein an output of each of the plurality of artificial neurons is computed based on inputs of the artificial neuron in accordance with a plurality of weights.

In these implementations, the actions include processing, by the data processing system, new data using the plurality of artificial neurons in the trained neural network in accordance with the values of the plurality of weights to identify the one or more near real-time predictions of enrollment for the given clinical trial, wherein the artificial neurons in the input layer are configured to receive the new data as input and the artificial neurons in the output layer are configured to generate a new output that identifies a near real-time prediction of enrollment, and wherein the new data includes the target schema and the received input data. The actions include training the neural network to predict enrollment, including: receiving training data, with one or more portions of the training data being associated with a label specifying an enrollment count; defining a first squared error function that quantifies a prediction error of the neural network based on the training data; and setting weights of the neural network to minimize the first prediction error.

In these implementations, the neural network is a recurrent neural network. The one or more visualizations provide a first view at a study level or a second view at a country level. The actions further include: determining that at least one or more near real-time predictions specifies enrollment that is less than a threshold amount; responsive to the determining, generating functional data that when received by a clinical trial management system causes the clinical trial management system to perform one or more operations, wherein at least one of the one or more operations is allocating memory and processing resources for generating one or more communications and transmitting over a network the one or more communications to one or more client devices, wherein each client device is associated with a site of the clinical trial study and a communication informs a user of a client device that has received the communication that the enrollment is less than a threshold amount and may prompt the user to perform one or more actions, and transmitting the functional data to a clinical trial management system. The functional data is an alert.

In still other implementations, one or more machine-readable hardware storage devices for generating a target schema for use with a neural network to generate output indicative of a near real-time prediction of clinical study enrollment, the one or more machine-readable hardware storage devices storing instructions that are executable by one or more processors to perform operations including: causing rendering of one or more user interfaces with one or more input portions for input of data specifying a clinical trial; receiving, from the one or more user interfaces, input data corresponding to data input into the one or more portions of the user interface, with the input data received specifying a given clinical trial; based on the received input data, generating, by a data processing system, a target schema for the given clinical trial, with the target schema specifying a target enrollment and a completion date for the given clinical trial; accessing, from one or more hardware storage devices, historical data and enrollment data for the given clinical trial, with the historical data being from one or more studies conducted in a disease area associated with the clinical trial, and with the enrollment data specifying actual enrollment in the clinical trial; inputting, into a neural network, the target schema, the enrollment data and the historical data; receiving, from the neural network, prediction data specifying one or more near real-time predictions of enrollment for the clinical trial; and outputting to one or more user interfaces one or more visualizations of the received prediction data.

In these implementations, the clinical trial is associated with one or more sites, wherein the accessed enrollment data is for a given one of the one or more sites, and wherein the prediction data specifies one or more near real-time predictions of enrollment for the clinical trial for the given one of the one or more sites. The target schema specifies an enrollment target, an activation date and/or a completion date. The operations further include: calibrating the neural network by: accessing, from a hardware storage device, clinical trial data; segmenting the clinical trial data into test data and training data; for the training data, identifying one or more features of the training data; encoding the one or more features; transmitting at least a portion of the one or more encoded features to one or more hidden layers of the neural network; based on an output of the one or more hidden layers, determining one or more values of one or more hyperparameters of the neural network; and repeating the transmitting and the determining until the neural network outputs one or more values satisfying one or more thresholds.

Other embodiments of the foregoing aspects include corresponding computer systems (e.g., a data processing system), apparatus, and computer programs recorded on one or more computer storage devices, each configured to perform the actions or operations described herein. A system of one or more computers can be configured to perform particular actions by virtue of having software, firmware, hardware, or a combination of them installed on the system that, in operation, causes or cause the system to perform the actions. One or more computer programs can be configured to perform particular actions by virtue of including instructions that, when executed by a data processing apparatus, cause the apparatus to perform the actions.

In the fast-paced world of clinical trials, real-time observation of ongoing studies is of utmost importance. This continuous monitoring allows researchers to stay abreast of the study's progress, identify potential bottlenecks or areas of concern, and take timely action to ensure that study objectives are met. As these objectives often include specific enrollment targets within specified timeframes, it's crucial to have an accurate and dynamic tool to predict future enrollments based on the data accrued throughout the study. As such, the primary field of this technology is the use of a machine learning model (gated recurrent unit (GRU) based recurrent neural network (RNN)) with the combination of historical clinical trial enrollment data and ongoing clinical trial's actual patient enrollments observed to-date to generate a study's future enrollment predictions. Customers are provided with enrollment predictions on a per site basis, along with aggregated study and country level predictions to assess the study enrollment progress and to identify potential countries or sites that require intervention.

Other solutions on study enrollment tracking and monitoring can be insufficient in the following aspects. First, alternative solutions are not integrated with a system that tracks study patient enrollment. The system described herein is integrated with a direct data feed of ongoing study subject enrollment, e.g., via an electronic data capture system (EDC) or a clinical trial management system (CTMS). Second, alternative solutions only provide tracking / monitoring functionalities. Users can only see the study's enrollment to-date and use their own judgment on whether the study will still be able to meet enrollment targets on time. Or the alternative systems require users to input their own forecasts or projections of future enrollment. The system described herein automatically generates a predicted enrollment trajectory based on actual enrollment to-date and historical data.

Third, alternative solutions utilize an extrapolation enrollment prediction approach that lacks accuracy. For example, enrollment forecasting that is simply an extrapolation method based on observed enrollment rates to-date lacks accuracy. In validation exercises performed using the techniques described herein, the techniques described herein had an accuracy improvement between 20% to 50% over such simplistic extrapolation methods.

The techniques described herein solve the technical problem of accurately predicting a clinical trial's future enrollment as the study progresses and provides the flexibility to generate an updated prediction at various time points for constant monitoring purposes. The techniques described herein provide for both an aggregated view at the study level and the ability to zoom in to specify participating countries or sites for diagnostic analyses. Depending on the specific study design, users can apply various customizations to predictions such as specifying a site's start-up activation time or denoting a site as closed for enrollment once a certain number of patients have been enrolled.

The techniques described herein utilize a data-driven, deep learning model that predicts the future number of patients enrolled at a given site in a given study, for each month up until a user specified maximum number of months. The granularity of the prediction (study-site-month) allows the prediction result to be viewed by a user in both an aggregated study view and in more detailed per-country and per-site views. RNNs are utilized, as they are best suited for sequence generation. This methodology allows for the flexibility to generate a prediction at various time points while the study progresses. The data processing system described herein combines historical data of past studies conducted within the disease area of research and historical data of the participating countries/sites, and the actual enrollments observed to-date as inputs to generating the predictions; thus the model can learn from historical patterns and also adjust for any specifics within the ongoing study (e.g., rare patient populations, restrictive study eligibility criteria, and different macro-environment context ). The model performance has been validated on a test set with over 2,000 studies and superior accuracy against various baseline methodologies has been demonstrated. Furthermore, several pre-processing and post-processing algorithms are developed to handle the potential user customization needs as mentioned herein.

The techniques described herein provide numerous advantages, including that the system integrates with an EDC and/or CTMS data as the source of actual enrollment data on a periodic data refresh. This system automatically generates an up-to-date predicted enrollment trajectory whenever users request an updated forecast based on the most recently received available data. Users can compare the predicted trajectory of a study enrollment timeline relative to their planned time and determine whether any intervention is required to meet targeted study enrollment timelines. The predictions are based on both historical data from studies conducted within the same disease area and adjusted for the observed actual enrollment behavior of each specific study. Utilization of deep learning models provides the flexibility of generating predictions at various time points as well as improved accuracy of the predictions. The model was validated on a large test set of clinical trials and has demonstrated both superior accuracy and generalizability.

The details of one or more embodiments of the subject matter of this specification are set forth in the accompanying drawings and the description below. Other features, aspects, and advantages of the subject matter will become apparent from the description, the drawings, and the claims.

### BRIEF DESCRIPTION OF THE DRAWINGS

FIG. 1A shows a software development environment for calibrating a neural network to output near real-time predictions of clinical trial study enrollment.
FIG. 1B shows a data processing system for controlling operations of a clinical trial management system.
FIG. 2 shows an example data processing system for using a neural network to output near real-time predictions of clinical trial study enrollment.
FIG. 3 shows an example calibration.
FIG. 4 depicts an example of training a neural network.
FIG. 5 depicts an example system workflow.
FIG. 6 depicts example system inputs and outputs.
FIGS. 7A-7C each shows an example graphical user for adding and/or editing a study including site attributes.
FIG. 8A shows an example graphical user interface for viewing an aggregated study enrollment forecast.
FIG. 8B shows an example graphical user interface for viewing country level predictions.
FIG. 8C shows an example graphical user interface for viewing site level predictions.
FIG. 9 shows an example process to develop and validate a study enrollment forecasting model.
FIG. 10 illustrates the model performance against baselines.
FIG. 11 shows an example process for generating a target schema for use with a neural network to generate output indicative of a near real-time prediction of clinical study enrollment.
FIG. 12 depicts an example computing system, according to implementations of the present disclosure.

### DETAILED DESCRIPTION

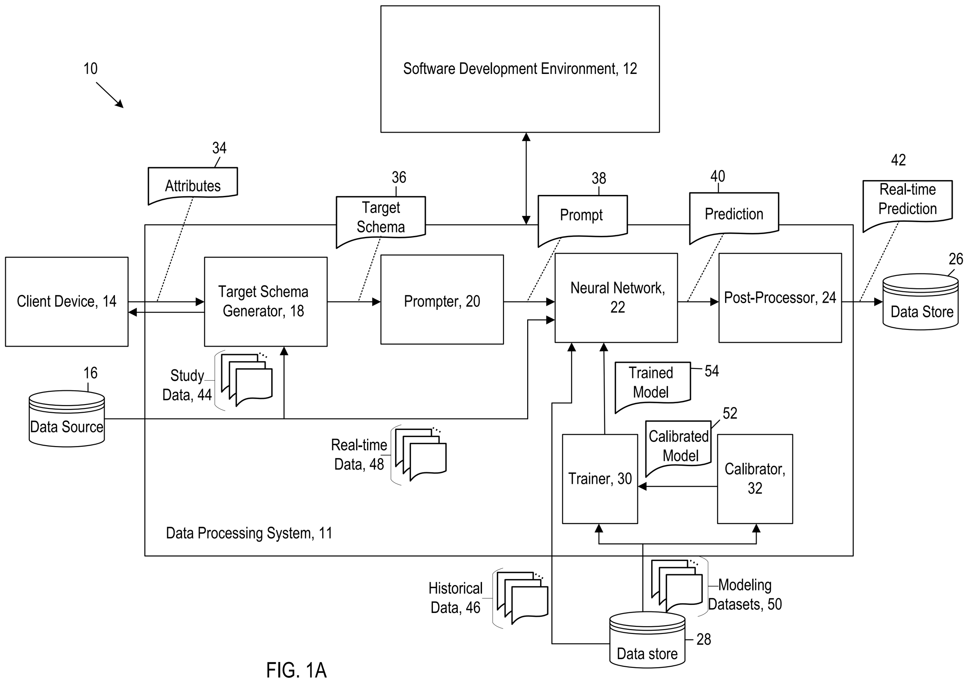

Referring to FIG. 1A, data processing environment 10 is shown. Data processing environment 10 includes software development environment 12 for developing and programming software. Software development environment 12 includes various software development tools and APIs, for example, to enable the writing of software to perform the functionality of data processing system 11, as described herein. That is, software development environment 12 includes a workspace with a set of processes and programming tools used to develop the source code for an application or software product. Data processing environment 10 includes client device 14 for transmitting data to data processing system 11 and for receiving data from data processing system 11. Data processing environment 10 also includes data source 16 for transmitting data to data processing system 11 as described herein. Data source 16 can be a CTMS, an EDC, and so forth. In this example, data source 16 is integrated with data processing environment 10 to enable real-time data 48 to be automatically retrieved from data source 16.

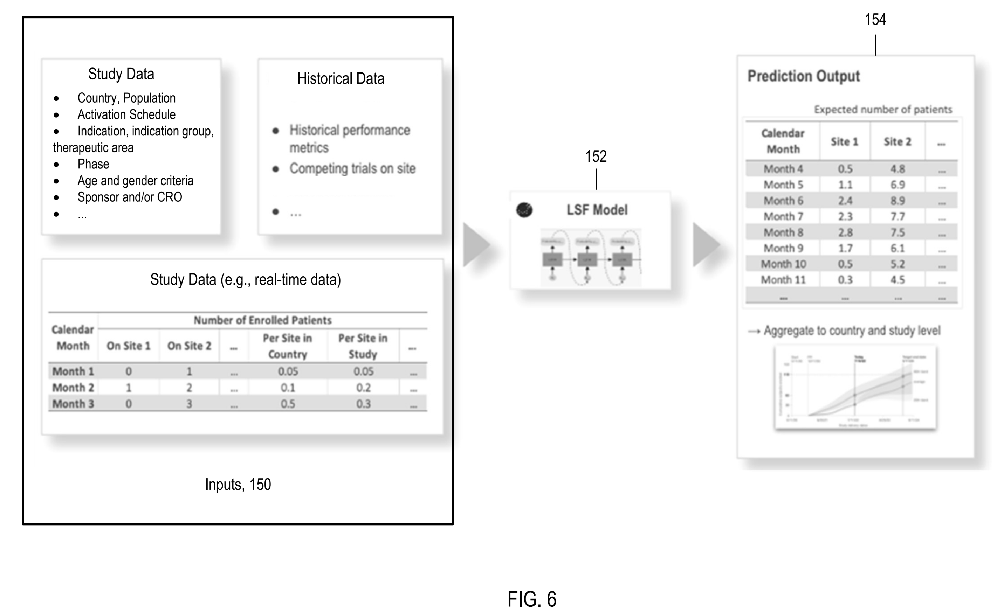

Data processing system 11 includes target schema generator 18 for generating a target schema. Generally, a target schema includes data of a specified type, data of a specified format, data including specified contents (e.g., values), and/or a data structure that is structured with fields for storing specified types of data. For example, a target schema may specify a particular study (or a particular site within a study), a target enrollment and a completion date (e.g., when the target schema is at a study level) and an activation date (e.g., when the target schema is at a site level). In this example, data source 16 transmits study data 44 to target schema generator 18. Study data 44 specifies available studies for which enrollment data can be forecasted. Study data 44 includes information about the studies (e.g., clinical trial studies) such as start date, end date, type of study, target enrollment, and the geographic locations of one or more sites for the study. Study data 44 also includes real-time data, e.g., real-time data specifying actual enrollment performance (e.g., as shown in FIG. 6). Using study data 44, target schema generator 18 generates graphical user interface data that when rendered on client device 14 displays one or more graphical user interfaces for selecting a particular study for which enrollment is forecasted. Target schema generator 18 transmits this graphical user interface data to client device 14. In response, client device 14 renders the one or more graphical user interfaces and receives through the one or more graphical user interfaces input data specifying selection or otherwise indicating a particular study for which enrollment is to be predicted. Through the one or more user interfaces, a user is able to add or edit the study and site attributes as needed, for example by specifying study enrollment target, a site activation and/or a completion date. In this example, study enrollment target is a study attribute. Generally, site attributes specify characteristics of a particular site (e.g., geographic location) at which a clinical trial is being conducted. There are various types of site attributes, such as an activation date, a completion date, a country, a population (e.g., the population of that country for that site), and historical performance metrics and competing trials on site, for example. Some site attributes are part of study data and some site attributes are part of historical data. For example, country, population and activation date are part of study data. Historical performance metrics and competing trials on site are part of historical data.

Client device 14 transmits attribute data 34 to target schema generator 18. Attribute data 34 specifies various attributes of the selected study. These attributes include, for example, target completion date. Using attribute data 34, target schema generator 18 generates the target schema 36. In this example, attribute data 34 only specifies the study enrollment target. As such, to generate target schema 36, target schema generator 18 retrieves from study data 44 other pertinent information that is required for generation of target schema 36. This other pertinent information includes for example a study activation date/study start date and study completion dates. Target schema 36 also includes information specifying a particular site for the study. Target schema generator 18 transmits target schema 36 to prompter 20. In turn, prompter 20 generates prompt 38 and transmits prompt 38 to neural network 22. Prompt 38, for example, includes contents specifying that a predicted enrollment via a particular date is requested and also provides the target schema 36, thereby providing neural network 22 with the information that is needed to make a prediction of enrollment for a particular site for a particular clinical study by a target date.

Responsive to prompt 38, neural network 22 generates prediction data 40. In this example, prediction data 40 specifies a predicted enrollment count by a target date for a particular clinical site at a particular location. Neural network 22 transmits prediction data 40 to post-processor 24. Post processor 24 provides for execution of various processing operations on prediction data 40, including, for example, generating relevant key performance indicators (KPIs) and various visualizations representing the predicted enrollment. Post-processor 24 outputs real-time prediction 42, which includes data specifying customizations that are performed on prediction data 40. As explained herein, these customizations include formatting of the data, generation of visualizations to provide indications of the predicted enrollment, and so forth. Data store 26 receives real-time prediction data 42 from post-processor 24 and stores real-time prediction data 42.

In generating prediction data 40, neural network 22 also receives real-time data 48 from data source 16 and historical data 46 from data store 28. Historical data 46 specifies historical enrollments for a given clinical trial study, a given site and/or historical enrollments for analogous or similar sites. Historical data 46 can also specify enrollments for clinical trial studies in a same or similar disease area as the clinical trial study for which the prediction is being generated. For this to be accomplished, items of study data 44 specify a disease area, thereby enabling neural network 22 to retrieve historical data in an associated (e.g., same or similar) disease area. Real-time data 48 includes data specifying actual enrollments to-date for the particular study at the particular site. Providing neural network 22 with historical data 46 and real-time data 48 enables neural network 22 to provide a more accurate prediction. Generally, real-time data 48 includes data that is received by data processing system 11 in real-time, near real-time or live time relative to when the data is originally received by data source 16. Generally, near real-time pertains to the timeliness of data or information which has been delayed by the time required for electronic communication and automatic data processing. Usage of the term real-time herein includes real-time and/or near real-time.

In this example, data processing system 11 also includes trainer 30, calibrator 32 and data store 28. Calibrator 32 is for tuning a neural network and for determining hyper parameters of that neural network. Once the neural network is finely tuned, trainer 30 then uses training data to train the neural network. In this example, data store 28 transmits modeling datasets 50 to trainer 30 and calibrator 32. Modeling datasets 50 includes trial level and trial site level data for various clinical trials. Calibrator 32 uses modeling datasets 50 to calibrate or fine-tune the neural network, as will be described in detail below. Calibrator 32 outputs calibrated model 52 to trainer 30. Trainer 30 then uses modeling datasets 50 to train calibrated model 52 to produce trained model 54, as will be described in detail herein. Trainer 30 outputs trained model 54 to neural network 22. In this example, neural network 22 is/or includes trained model 54.

In a variation of FIG. 1A, neural network 22 could more generally be a machine learning model. There are various types of machine learning models, including, e.g., a generative artificial intelligence (AI) model having one or more large language models (LLMs). Example LLMs include models having one or more generative pre-trained transformers (GPTs), such as those implemented using one or more artificial neural networks.

Generally, machine learning can encompass a wide variety of different techniques that are used to train a machine to perform specific tasks without being specifically programmed to perform those tasks. The machine can be trained using different machine learning techniques, including, for example, supervised learning, unsupervised learning, and reinforcement learning. In supervised learning, inputs and corresponding outputs of interest are provided to the machine. The machine adjusts its functions in order to provide the desired output when the inputs are provided. Supervised learning is generally used to teach a computer to solve problems which are outcome determinative, for example, the training set may be used to train the machine learning model. In contrast, in unsupervised learning, inputs are provided without providing a corresponding desired output. Reinforcement learning describes an algorithm in which a machine makes decisions using trial and error. Feedback informs the machine when a good choice or bad choice is made. The machine then adjusts its algorithms accordingly. For example, the trained learning model may be embodied as a generalized linear model (GLM). Different types of generalized linear models may be appropriate in various scenarios. A zero-inflated negative binomial generalized linear regression may be used because it models a discrete count of events (such as conversions) occurring in a given time period.

In another example, the trained machine learning model may be embodied as an artificial neural network, as shown in FIG. 1A. Artificial neural networks (ANNs) or connectionist systems are computing systems inspired by the biological neural networks that constitute animal brains. An ANN is based on a collection of connected units or nodes, called artificial neurons. Each connection, like the synapses in a biological brain, can transmit a signal from one artificial neuron to another. An artificial neuron that receives a signal can process it and then signal additional artificial neurons connected to it.

In common ANN implementations, the signal at a connection between artificial neurons is a real number, and the output of each artificial neuron is computed by some non-linear function of the sum of its inputs. The connections between artificial neurons are called 'edges'. Artificial neurons and edges may have a weight that adjusts as learning proceeds (for example, each input to an artificial neuron may be separately weighted). The weight increases or decreases the strength of the signal at a connection. Artificial neurons may have a threshold such that the signal is only sent if the aggregate signal crosses that threshold. The transfer functions along the edges usually have a sigmoid shape, but they may also take the form of other nonlinear functions, piecewise linear functions, or step functions. Typically, artificial neurons are aggregated into layers. Different layers may perform different kinds of transformations on their inputs. Signals travel from the first layer (e.g., the input layer), to the last layer (e.g., the output layer), possibly after traversing the layers multiple times.

In general, a neural network is trained using a supervised learning technique based on training data, wherein the neural network is configured to receive training data as input (e.g., input data records) and to process the input to generate an output, e.g., that specifies whether PII is detected. In this example, the neural network includes a plurality of artificial neurons that are connected through edges and are aggregated into a plurality of neural network layers including at least an input layer and an output layer, wherein each of the edges is configured to transmit a signal from one artificial neuron to another artificial neuron, and wherein an output of each of the plurality of artificial neurons is computed based on inputs of the artificial neuron in accordance with a plurality of weights. In this example, new data is processed using the plurality of artificial neurons in the trained neural network in accordance with the values of the plurality of weights to detect PII, wherein the artificial neurons in the input layer are configured to receive the new data as input and the artificial neurons in the output layer are configured to generate a new output that specifies whether PII is detected.

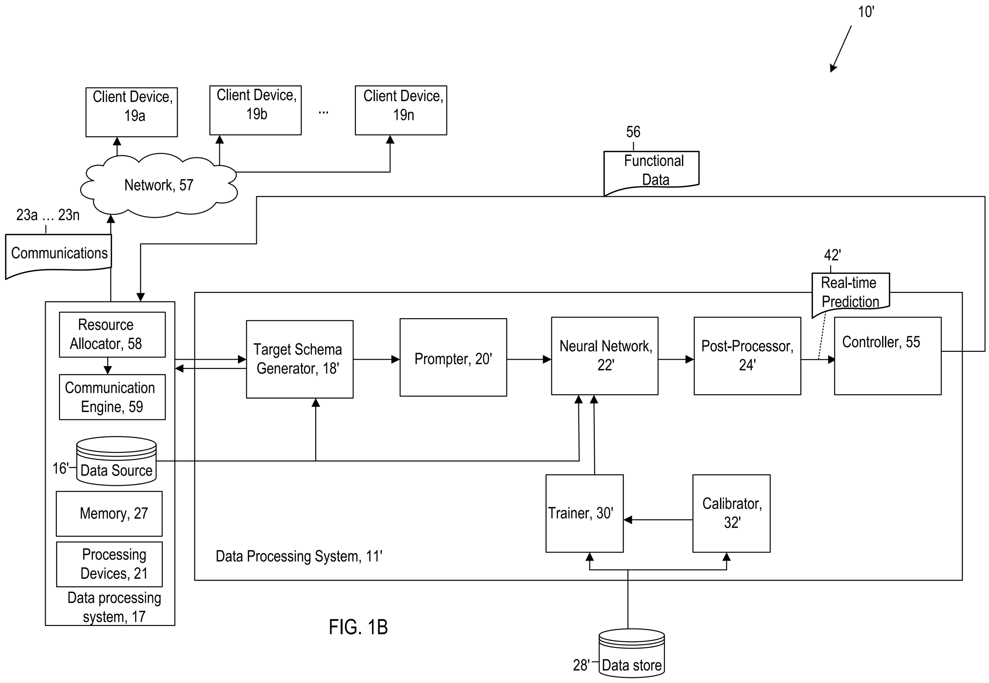

Referring to FIG. 1B, data processing environment 10' is shown. Data processing environment 10' is a version of data processing environment 10 (FIG. 1A). Data processing environment 10' includes data processing system 11' (which is the same as or a version of data processing system 11 in FIG. 1A), with target schema generator 18' (which is the same as or a version of target schema generator 18 in FIG. 1A), prompter 20' (which is the same as or a version of prompter 20 in FIG. 1A), neural network 22' (which is the same as or a version of neural network 22 in FIG. 1A), post-processor 24' (which is the same as or a version of post-processor 24 in FIG. 1A), trainer 30' (which is the same as or a version of trainer 30 in FIG. 1A) and calibrator 32' (which is the same as or a version of calibrator 32 in FIG. 1A). Data processing environment 10' also includes data store 28' (which is the same as or a version of data store 28 in FIG. 1A) and data processing system 17. Data processing environment 10' includes network 57 and client devices 19a ... 19n.

Data processing system 11' includes controller 55 that is pre-programmed with logic to detect when a predicted enrollment (specified by real-time prediction 42') is below a threshold or specified amount. In this example, post-processor 24' transmits to controller 55 information specifying the target enrollment, which is the threshold or specified amount. When controller 55 detects that the amount specified by real-time prediction 42' is less than the target amount, controller 55 generates functional data 56, which controls an operation of data processing system 17 and causes data processing system 17 to perform one or more operations, as described below. In some examples, functional data 56 includes an alert specifying that an enrollment specified by real-time prediction 42' is less than the target enrollment.

Data processing system 17 includes data source 16', processing devices 21, memory 27, resource allocator 58, and communication engine 59. Data source 16' is the same as or a version of data source 16 in FIG. 1. Resource allocator 58 includes logic to allocate the memory and processing resources of data processing system 17, e.g., based on the various tasks that data processing system 17 needs to perform. In this example, the memory resources are memory 27 and/or data source 16'. The processing resources are processing devices 21. Communication engine 59 is programmed with logic to generate and transmit communications to external systems, e.g., client devices 19a ... 19n.

Data processing system 17 runs both when the predicted enrollment will not meet the target enrollment and when the predicted enrollment will exceed the target enrollment. In this example, data processing system 17 receives functional data 56. Based on contents of functional data 56, data processing system 17 determines that the predicted enrollment will not meet the target enrollment. When this occurs, data processing system 17 is programmed with logic to perform the following operations: data processing system 17 notifies communication engine 59 that communications need to be sent to client devices 19a...19n. In this example, a client device is associated with a site of the clinical trial study and a communication informs a user of a client device that has received the communication that the enrollment is less than a threshold amount and may prompt the user to perform one or more actions. Data processing system 17 also notifies resource allocator 58 that communication engine 59 needs to generate and transmit these communications, and requests that resource allocator 58 make available sufficient processing and memory resources for the communications to be generated and transmitted. In this example, resource allocator 58 is programmed with logic specifying how much processing resources and memory resources communication engine 59 requires to generate and to transmit the communications. By executing this programmed logic, resource allocator 58 determines whether it needs to shut, pause or delay other tasks and/or operations being performed by data processing system 17 to ensure that there are sufficient memory and processing resources available for generation and transmission of the communication.

In this example, once resource allocator 58 has determined that there are sufficient memory and processing resources available, resource allocator 58 notifies communication engine 59. In turn, communication engine 59 generates communications 23a ...23n and transmits communications 23a ...23n to client devices 19a ...19n to prompt user or client devices 19a ... 19n to perform one or more actions.

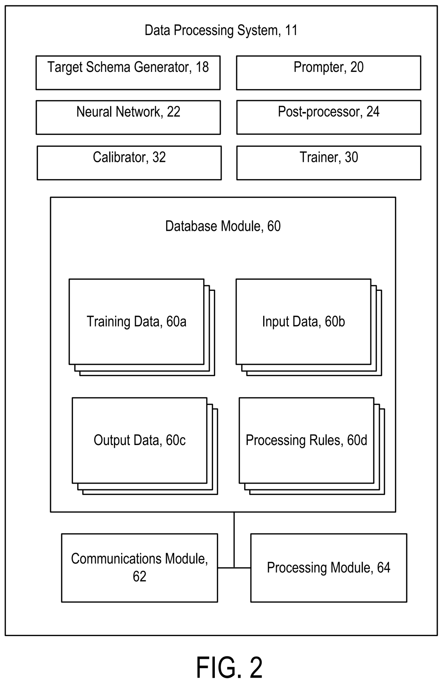

FIG. 2 shows various aspects of the data processing system 11. In general, the data processing system 11 includes several operation modules that perform particular functions related to the operation of the data processing system 11. For example, the data processing system 11 includes target schema generator 18, prompter 20, neural network 22, post-processor 24, trainer 30 and calibrator 32, as previously described. Further, the data processing system 11 includes a database module 60, a communications module 62, and a processing module 64. The operation modules can be provided as one or more computer executable software modules, hardware modules, or a combination thereof. For example, one or more of the operation modules can be implemented as blocks of software code with instructions that cause one or more processors of the data processing system 11 to execute operations described herein. In addition, or alternatively, one or more of the operation modules can be implemented in electronic circuitry such as, e.g., programmable logic circuits, field programmable logic arrays (FPGA), or application specific integrated circuits (ASIC).

The database module 60 maintains information related to real-time enrollment predictions using neural network 22. As an example, the database module 60 can store training data 60a for training or prompting the neural network 22. In some implementations, the training data 60a can include data representing historical clinical trial studies and an associated enrollment count for that clinical trial study. The data representing a clinical trial study can specify attributes of the clinical trial study that can also specify a disease area studied by (or otherwise associated with) the clinical trial study. Such attributes can include name, sites of the clinical trial study, start date, completion date, target enrollment, and so forth.

As another example, the database module 60 can store input data 60b that is used as an input to neural network 22. As an example, the input data 60b can include commands or instructions provided by a user, including information regarding a particular desired output of the data processing system 11, information input into a graphical user interface, and so forth. For instance, the input data 60b can specify a particular clinical trial study for which an enrollment prediction is requested. The input data 60b can also include a target enrollment, start date, completion data and information about individual sites that are participating in the clinical trial study, as well as a disease area of the clinical trial study, and/or any other information regarding the clinical trial study.

Further, the input data 60b can include historical data, e.g., site historical performance metrics that are derived from historical studies. The historical studies include start date, completion date, actual enrollment per month, and so forth. The input data 60b also includes real-time enrollment data for the clinical trial study (and/or for a particular site of the clinical trial study) for which a prediction is generated. In this example, a computer system (e.g., associated with the clinical trial study) transmits to data store 16 (FIG. 1A) information specifying an enrollment count at scheduled times, at periodic times, intermittently, continuous and so forth.

The database module 60 can also store output data 60c generated by neural network 22. As an example, the output data 60c can include near real-time predictions of enrollment for a particular clinical trial study and/or for a particular site of a particular clinical trial study. For example, the output data 60c can be used to generate site predictions, which are then aggregated into study predictions.

Further, the database module 60 can store processing rules 60d (e.g., rules specifying site historical performance metrics to retrieve and/or a type of other historical data to retrieve, including, e.g., historical data associated with a same disease area as the clinical trial study for which the near real-time prediction is generated) specifying how data in the database module 60 can be processed to generate a near real-time enrollment prediction using neural network 22 and/or which data to retrieve from one or more data stores. As discussed with regard to FIG. 6, historical data also includes data specifying competing trials on site.

In another example, the processing rules 60d can include one or more rules for implementing, instruction tuning or prompting, and operating neural network 22 to produce the output data 60c. For example, the one or more rules can specify that the training data 60a be provided to neural network 22 for training or prompting (e.g., such that neural network 22 can generate enrollment predictions, identify trends and/or correlations between the contents of clinical trial input data and the subject matter therein, and generate new output based on those identified trends and/or correlations). As another example, the one or more rules can specify that the input data 60b be provided to neural network 22 (e.g., to generate output data 60c representing an enrollment prediction). As another example, the one or more rules can specify that the generated output data 60c be presented to the user and/or stored for future retrieval and/or processing (e.g., using the database module 60). As another example, the one or more rules can specify one or more tools that facilitate the performance of particular actions by neural network 22. For example, the tools can specify certain actions or operations that can be performed by neural network 22 to generate an enrollment prediction, retrieve data and generate content based on the retrieved data, and so forth. Example data processing techniques are described in further herein.

As described above, the data processing system 11 also includes a communications module 62. The communications module 62 allows for the transmission of data to and from the data processing system 11. For example, the communications module 62 can be communicatively connected to a network, such that it can transmit data to and receive data from the client device 14 (FIG. 1A). Information received from the client device 14 can be processed (e.g., using the processing module 64) and stored (e.g., using the database module 60).

As described above, the data processing system 11 also includes a processing module 64. The processing module 64 processes data stored or otherwise accessible to the data processing system 11. For instance, the processing module 64 can be used to execute one or more of the operations described herein (e.g., operations associated with neural network 22).

In some implementations, a software application can be used to facilitate performance of the tasks described herein. As an example, an application can be installed on the data processing system 11 and/or client device 14. Further, a user can interact with the application to input data and/or commands to the data processing system 11, and review data generated by the data processing system 11.

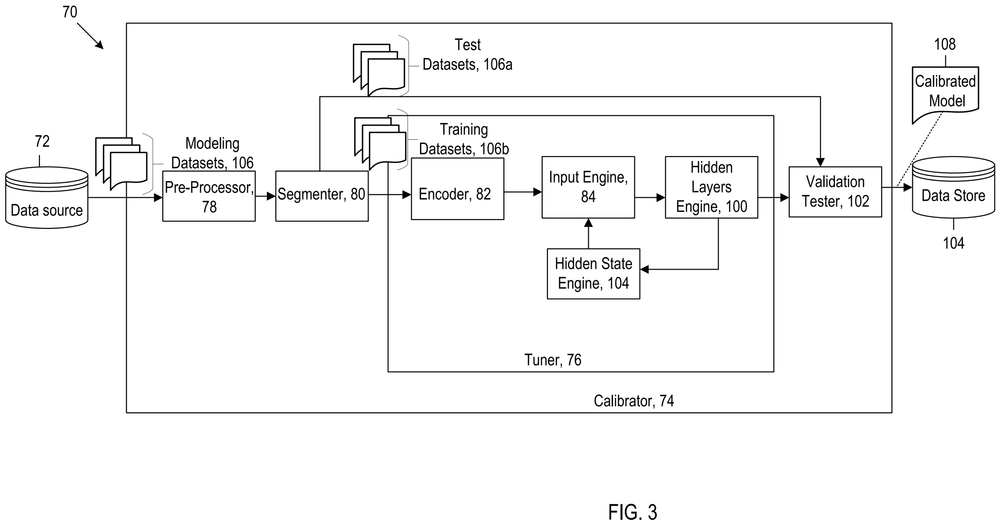

Referring to FIG. 3, calibration environment 70 is shown. Calibration environment 70 includes calibrator 74 for calibrating or fine-tuning a neural network (e.g., a recurrent neural network). Calibration environment 70 also includes data source 72 and data store 104. In operation, data source 72 sends or transmits modeling datasets 106 to pre-processor 78. Modeling datasets 106 include trial level data and trial site level data. Data source 72 transmits modeling datasets 106 to pre-processor 78 which organizes the data in the correct sequence and converts it to accurately reflect calendar months, for example. In the context of data pre-processing, it's crucial to preserve sequential data order because current neural networks are designed to retain an internal state of memory that processes input sequences, capturing temporal dependencies and patterns overtime, as discussed in further detail below.

Pre-processor 78 outputs the pre-processed data to segmenter 80, which splits the pre-processed data into test datasets 106a and training datasets 106b, as will be explained herein. Calibrator 74 includes tuner 76 for determining values (optimal values) of hyper parameters of the neural network. In this example, tuner 76 includes encoder 82. Segmenter 80 transmits training datasets 106b to encoder 82. Encoder 82 performs various types of encoding on training datasets 106b, for example, performing one hot encoding. One hot encoding is a method for converting categorical variables into a binary format. The primary purpose of one hot encoding is to ensure that categorical data can be effectively used in machine learning models. Encoder 82 transmits the encoded training datasets to input engine 84.

In this example, input engine 84 acts as an input layer in the neural network for receiving the information that is going to be used for fine-tuning the neural network. The input engine 84 transmits the encoded training datasets to hidden layers engine 100 which starts a forward pass through a RNN as described herein. At each step, the RNN takes in two inputs: the current input, which is received from input engine 84 and a hidden state from the previous time step. The RNN performs computation on these inputs to produce a new hidden state, which is produced by hidden state engine 104. The new hidden state is then passed on to the next time step (e.g., is received by input engine 84) and this process continues. Once conversions are reached during training, hidden layers engine 100 outputs a proposed tuned model to validation tester 102 which uses test datasets 106a to test the validity of the tune model. If the tuned model is of sufficient accuracy, then validation tester 102 outputs calibrated or tuned model 108 to data store 104. Additional details of the calibration environment are provided below.

Given the sequential nature of enrollment data, where each period is influenced by the ones preceding it, RNNs are ideally suited for this task. They can remember important information from past periods while incorporating new data, effectively learning from the enrollment history to predict future trends.

Practically, this means that if a study is falling behind its target enrollment trajectory, the RNN's model can identify this trend early, allowing stakeholders to take corrective actions promptly. Similarly, if a study is progressing faster than anticipated, resources can be re-allocated to ensure optimal efficiency. As such, the use of deep learning models such as RNNs provides a powerful tool to transform raw enrollment data into actionable insights.

In the realm of accurately predicting study enrollment at various stages, the complex, multidimensional and sequential dataset requires a deep learning model sophisticated enough to discern the underlying patterns and interdependencies. RNNs are designed to work with sequential data, which is inherent in clinical trial enrollment data. While traditional machine learning (ML) models view all input data as independent, RNNs can recognize patterns across time due to the "recurrent" nature. The 'recurrent' part of the RNN signifies its ability to remember or retain previous output in its internal memory while processing the current input. This ability allows an RNN unit to connect information from earlier steps to later ones as a sequence, making an ideal choice for processing large datasets with temporal elements.

Additionally, RNNs can simplify the process of feature engineering for time series data. Feature engineering is the process of transforming raw data into features that better represent the underlying problem, resulting in improved model performance. In the case of time-series data, traditionally, lagged versions of variables, trends, seasonality indicators and other temporal components are manually generated to provide a model with enough context. With RNN's inherent ability to process sequential data, they are capable of encoding information from previous data points, which eliminates the need to engineer these features.

Traditional RNN models have a significant limitation on the vanishing gradient problem which can be explained as the impact of a far past event that can become less and less noticeable, until it vanishes entirely. This limits the traditional RNN model's ability to process long sequences and retain the information from earlier stages of the sequence. GRUs are utilized to solve some of the challenges encountered with traditional RNNs, known as the 'vanishing gradient' problem. It means RNNs can struggle with connecting information from much earlier in the sequence with later elements. With the gated mechanism in GRUs, it controls the amount of internal state information that will be kept or forgotten to the next new element, making the model more capable of learning long-term dependencies.

The training data is composed of a mix of raw trial enrollment data and derived features, processed using the enrollment modeling (EM) data pipeline. This pipeline generates two primary streams of metrics: study site data and study site-month data. Study site data is focusing on descriptive details of each study site, including site capacity, past performance and attributes impacting patient enrollment. Study site-month data is focusing on a monthly snapshot, detailing enrollment rate, illuminating the evolving dynamic of a site over time.

In this example, a database (e.g., EDC 72 in FIG. 3) stores data from clinical trials spanning numerous decades, conducted across hundreds of countries, covering hundreds of indications and falling under numerous therapeutic areas.

Preprocessing input data (e.g., as performed by pre-processor 78) involves organizing data in the correct sequence and converting it to accurately reflect calendar months. In the context of data preprocessing, it's crucial to preserve sequential data order because RNNs are designed to retain an internal state or 'memory' that processes input sequences, capturing temporal dependencies and patterns over time - as previously described. By preserving the sequential nature of calendar months in the data, the RNN can effectively learn from past months to generate accurate predictions for future ones. The primary grain of data worked with is at the study-site level, and by using unique combinations of IDs (e.g., a DatabaseID, a StudyID, and a ClusterID (unique healthcare facility ID)), the techniques described herein precisely isolate features specific to the study site, the study itself, and the country level.

Each study-site has a rich feature set attached to it. This information includes both time-variant and constant features. Time-variant, or monthly variant features, encompass data that changes monthly and through aggregation, provides insights like the total number of patients recruited in each month during the study enrollment period within a country, the active study sites in that period, and the average number of patients per site at the country level. On the other hand, constant features examples include the study's indication, its therapeutic area, phase, and the associated sponsor, as well as country-specific data like population and density.

The preprocessed data is further split into a training set and a testing set to ensure the model (e.g., the neural network) can generalize to new, unseen data. Cross validation over the training set is used to conduct hyperparameter tuning and feature selection. The most common type is k-fold cross-validation, where the original sample is randomly partitioned into k equal sized subsamples. Of the k subsamples, a single subsample is retained as the validation data for testing the model, and the remaining k-1 subsamples are used as training data. In the data splitting process, each study is treated as a singular entity during sampling. This approach guarantees that the same study won't be partitioned across both training and testing sets. In the context of RNNs, it's vital to ensure that the temporal order of the data is preserved. The time-series cross-validation used respects this temporal order, allowing the model to learn meaningful temporal patterns during training that can then be tested on unseen future data.

Further for the training set, transformations are executed that are tailored to the nature of each feature type, including categorical, time-variant numerical, and constant numerical features. This approach ensures that the RNN can effectively learn patterns from the data. For the same features (e.g., categorical features), one-hot encoding is employed for its transformation. Other categorical features are initially converted to corresponding index format (e.g., to generate integer-coded categorical features). Embeddings are generated, e.g., by incorporating these integer-coded categorical features into distinct embedding layers before being passed to the RNN. An embedding layer essentially functions as a lookup table that maintains dense vectors for each category. That is, to incorporate a feature into an embedding layer, the feature is encoded as a categorical value (if it isn't already), then the encoded value is fed as input to the embedding layer; this allows the model to learn a vector representation of that feature, capturing relationships between different values of the feature within the embedding space during training. Utilizing an embedding layer for encoding categorical features offers several advantages, such as reduced dimensionality compared to one-hot encoding, and the ability to capture semantic relationships between different categories. Numerical features (e.g., population density) were z-standardized to aid in model training. In contrast, time-variant numerical features, such as the number of patient enrollments and number of sites activated, were retained in their original form, leveraging the RNN's inherent capability to handle time-series data.

After initializing the embedding layers, RNNs require inputs to maintain a consistent length for each batch. Due to sequences having varying lengths, padding is employed for shorter sequences, typically by adding zeros to their end, to match the length of the desired sequence. Conversely, sequences that are too long are truncated to ensure they conform to the specified length.

Before the forward pass through the two-layer RNN begins, the initial hidden state is set. This initial context for the RNN is created by passing the embeddings and numerical features through a series of fully connected linear layers. The forward pass through the RNN begins with the first "K" elements of the sequence, where 'K' represents the initial known segment of the data sequence. In this specific context, 'K' reflects the number of months since the study began its enrollment process. At each time step, the RNN unit takes in two inputs, the current input and the hidden state from the previous time step. The RNN performs computation on these inputs to produce a new hidden state. The new hidden state is then passed on to the next time step. The process is repeated in a loop beginning with the element at K+1 to the maximum length of the prediction. This process allows RNN to effectively process sequences and maintain information from previous time steps.

After processing, an activation function is applied to the raw output values of the RNN. Given the focus on predicting continuous, non-negative values, the ReLU (Rectified Linear Unit) activation function is employed at the output layer. ReLU effectively sets negative values to zero and allows positive values to remain unaltered. The inclusion of ReLU introduces non-linearity, helping the model to capture intricate patterns while maintaining computational efficiency.

During training, various techniques are employed to prevent overfitting. One such technique is dropout, which randomly sets a proportion of input units to 0 at each update during training time, which helps prevent overfitting. In addition to prevent overfitting (i.e., the model learning too much from the noise in the training data and not generalizing well to unseen data), noise is added directly to the embedding and other input to the model.

To promote faster convergence during training, the 'teacher forcing' technique is employed. Rather than consistently using the model's own prior predictions as input for the current time step, the actual ground truth value is occasionally inserted. This approach tends to accelerate the model's convergence, leading to more stable and precise models. The training process is carried out in iterations equal to the number of batches, which constitutes one epoch (i.e., a single complete pass of the entire training dataset through a learning algorithm). This process is performed on a cross-validation (CV) set, a subset of the training data, to ensure the model is not overfitting and generalizes well to unseen data.

Once the activations are generated, the RNN's outputs are matched with the actual values using a loss function. Following the computation of the loss, the model's parameters are updated. Backpropagation calculates the gradients of the loss with respect to the model's parameters, which are then used for the updates.

After each epoch, the performance of the model is assessed using the validation test set. This involves processing the validation data with the current model parameters, making predictions at different choices of K (1,3,8, etc.), where predicted values are compared with the actual values at the study-site level and the study level. This involves calculating the absolute difference between each predicted month and actual monthly enrollment values.

To optimize the performance of the model, hyperparameter tuning using the cross-validation set is executed. The tuning process leverages grid search, systematically exploring a range of hyperparameter values. This exhaustive search ensures a more thorough exploration, leading to optimal hyperparameter configurations.

Hyperparameters include the learning rate, dropout rate, and teacher forcing rate, along with the dimension of the embedding and linear layers, the number of layers in the RNN, the rate of L2-decay which is a regularization method that prevents overfitting by penalizing large weights, and the level of noise added to the input. The dimensions of the embedding and linear layers determine the complexity and capacity of the model to capture information. The number of layers in the RNN can affect its ability to capture complex patterns in the data. Noise addition to the input is a regularization technique which can improve generalization by making the model less sensitive to small changes in the input.

After selecting the best hyperparameters based on the cross validation set performance, the model is then retrained on the complete training set including the cross validation set. This process ensures that the model is tuned and ready for evaluation on unseen data.

Once the optimal hyperparameters are finely-tuned, the entire dataset is utilized for training. This approach ensures that the model has accessed the fullest extent of the available information, harnessing every bit of potential insight the data offers.

The model (e.g., the neural network) furnishes monthly enrollment predictions specific to individual study sites. Post processing can aggregate study level enrollment data at each calendar month. This granularity allows stakeholders to gain insights not just on a macro level, but also to understand the dynamics at individual study sites.

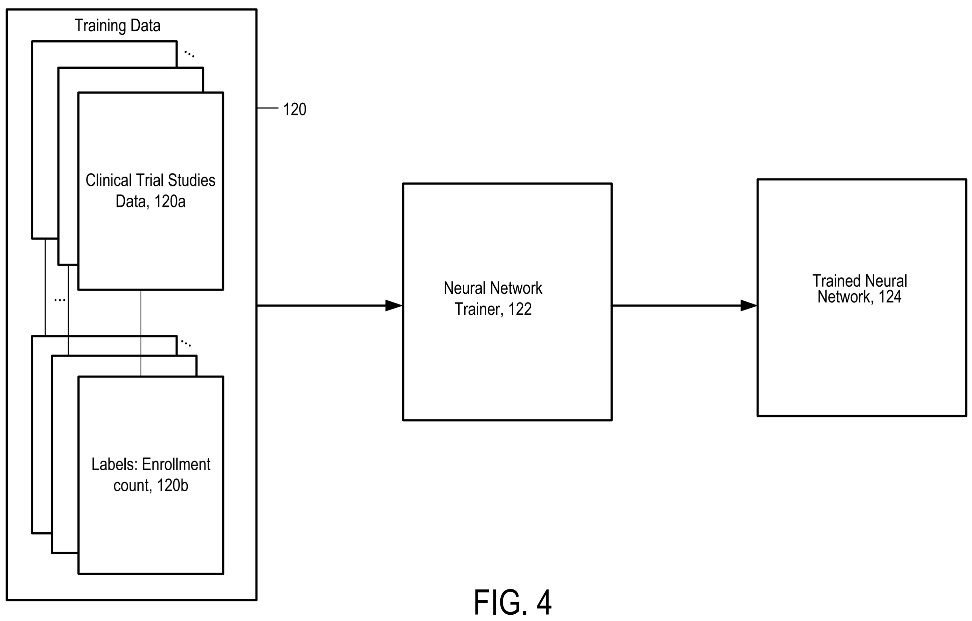

FIG. 4 illustrates an example of training a neural network. For example, training sets 120 input various types of input data (e.g., including numerical and categorical features). The training sets 120 may include clinical trial studies data 120a describing a clinical trial study (including, e.g., attributes of the clinical trial study and a disease area of the clinical trial study, numerical and categorical features, and so forth). The clinical trial studies data 120 is mapped via mapping structures to labels 120b. A mapping structure includes a pointer or other type of structure that associates one item of data with another. The labels 120b specify an enrollment count for the clinical trial studies represented in the clinical trial studies data 120a.

The training sets 120 may include any suitable number of sets of training data. Each training example in the training sets 120 may include contextual data specifying a name and other attributes of a clinical trial study. The contextual data may also include information about a structure of the data, including, e.g., which field stores or otherwise indicates a disease area of the clinical trial study. The specification of this field assists the neural network in requesting and retrieving historical data associated with that disease area.

A neural network 124 may be trained, by the neural network trainer 122, to generate a real-time enrollment prediction. In some implementations, the neural network trainer 122 may calculate numerical representations of training data (e.g., in vector form) for machine training. In some implementations, the neural network training techniques include feature extraction to build different neurons within a neural network. One or more features may translate an enrollment target being more likely to be achieved. For example, a particular feature may correspond to achievement of a particular enrollment target, such that the strength of a present feature leads to a particular metric (e.g., indicative of the enrollment target being achieved) being determined for the corresponding events (e.g., events indicative of start date, completion data and enrollment target).

In some implementations, a single machine learning model (e.g., neural network) may be trained. In other implementations, multiple models may be trained based on training sets 120. Once trained, the trained neural network is capable of receiving and processing requests. For example, for given clinical trial study input data, the trained neural network is able to predict enrollment.

To fine-tune the neural network, a small, high-quality, and diverse training dataset is used. This strategy prevents overfitting while ensuring the model performs well across different scenarios. The training dataset includes a variety of enrollment counts to provide balanced training data. Using the prepared datasets (as described above), the neural network is fine-tuned through supervised learning, where the inputs include numerical values, categorical values, and so forth) and the labels indicate the enrollment count. For example, the numerical values include historical data, such as historical performance metrics, competing trials on site and real time data, and so forth. The categorical values include country (e.g., "KOR", "CHN"), indication (e.g., "684" following ICD9 code), phase (e.g., "Phase III", "Phase IV", "Phase II", "Phase I"), gender (e.g., "female", "male"), and so forth.

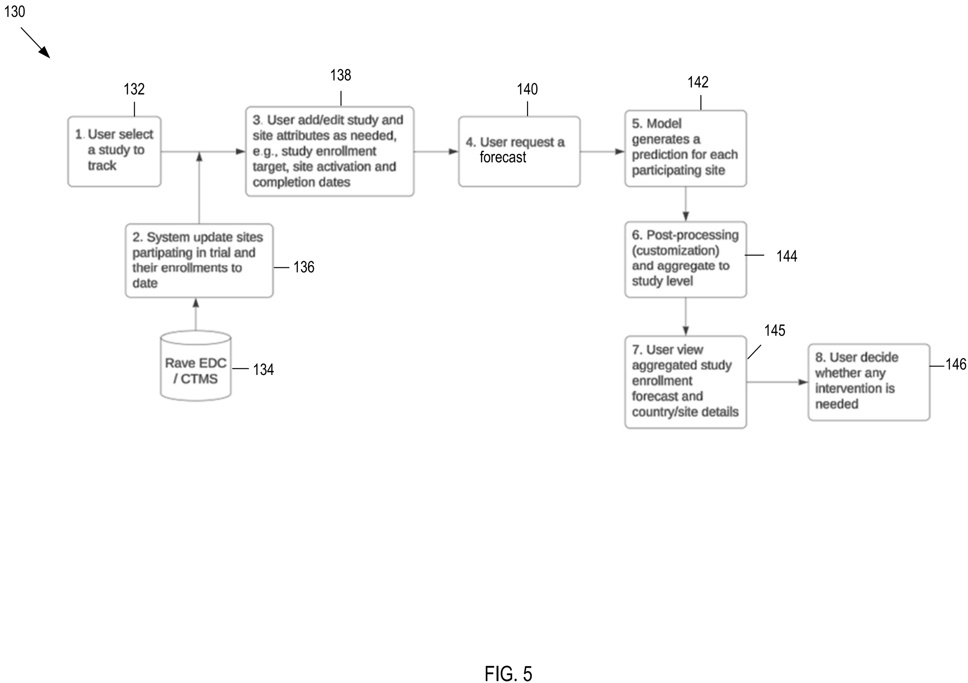

Referring to FIG. 5, system workflow 130 of the data processing system described herein is shown. In operation, EDC system 134 transmits to data processing system data specifying sites participating in a particular trial and their enrollments to-date. Using this information, the data processing system updates 136 sites participating in the trial and their enrollments to-date. Based on these updates, a user selects 132 a study to track and to predict enrollment data. A user is able to add/edit 138 the study and site attributes as needed, e.g., study enrollment target, site activation and completion dates. After the user has edited or modified the site attributes, the user requests 140 a forecast, e.g., to make a prediction of enrollment by a particular target date. The neural network model generates 142 a prediction for each participating site and/or for each selected site. The data processing system performs post processing 144 and also aggregates the data so that it is viewable at a study level and/or at a country level. The data processing system generates one more graphical user interfaces for a user to view 145 aggregated study enrollment forecasts and country details. Based on the visualizations in the graphical user interfaces, a user decides 146 whether any intervention is needed.

Referring to FIG. 6, example inputs and outputs are shown. In this example, inputs 150 include study data (including real-time data) and historical data. In this example, study data includes study attributes and site attributes and actual enrollment performance. Inputs 150 are input into neural network 152, which produces prediction output data 154. In this example, prediction output data 154 is broken down by month and is also broken down across various sites. Prediction output data 154 also includes an aggregation to look at predicted enrollment for a country level and a particular study level.

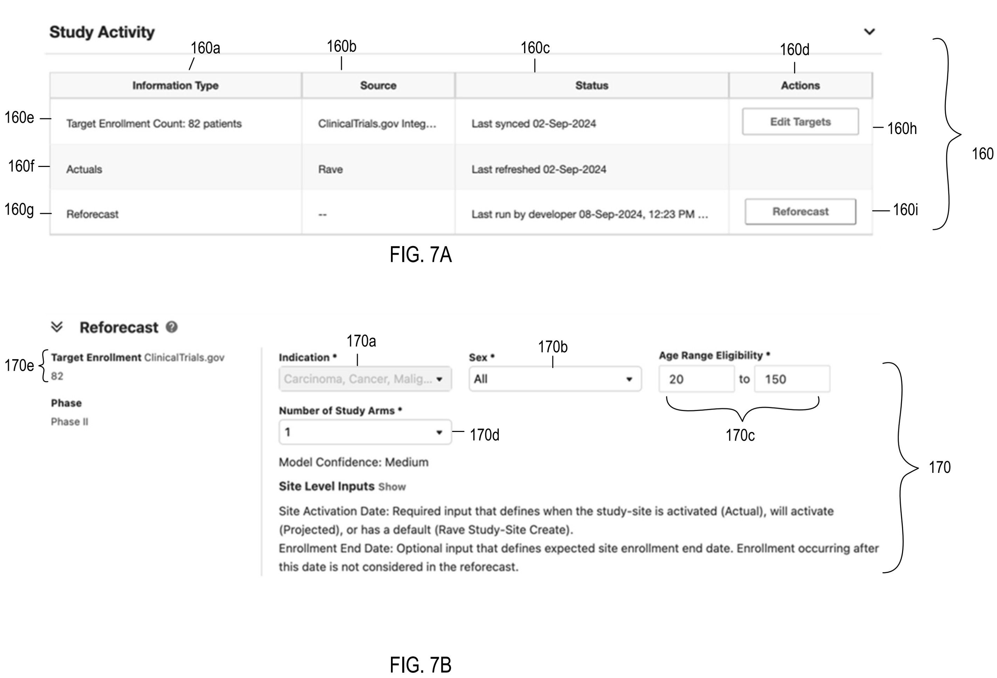

Referring to FIG. 7A, graphical user interface 160 is shown for enabling a user to add and/or edit a site and site attributes as needed. In this example, graphical user interface 160 includes a table with columns 160a, 160b, 160c and column 160d. The table also includes rows 160e, 160f, 160g. Column 160a specifies an information type such as, for example, a target enrollment. Column 160b specifies a source for that information type. Column 160c specifies a status for that information type. Column 160d specifies various actions that can be taken with regard to that information type. Rows 160e-160g specify the various information types. The actions specified by column 160d include controls 160h, 160i. Selection of control 160h enables a user to edit targets, such as target enrollments. Selection of control 160i causes the data processing system performed herein to update a prediction of enrollment by a target date.

Referring to FIG. 7B, graphical user interface 170 is shown for enabling a user to modify various attributes of a particular study. Graphical user interface includes control 170a that enables a user to modify a particular indication to be studied. Graphical user interface 170 includes control 170b that enables a user to specify a particular sex of patients to be included in the study for the forecast. Control 170c enables a user to specify an age range to be used in the forecast. Control 170d enables a user to specify a number of study arms to be used in the forecast. Graphical user interface 170 also includes portion 170e that specifies a target enrollment for the particular study.

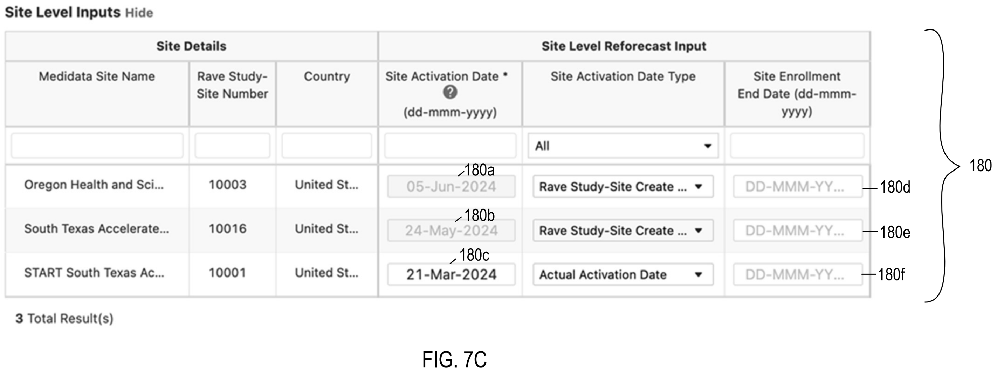

Referring to FIG. 7C, graphical user interface 180 is shown that enables a user to modify various attributes at the site level. In this example, graphical user interface 180 includes controls 180a-180c that enable a user to specify a site activation date. Graphical user interface 180 also includes controls 180d-180f that enables a user to specify a site enrollment end date.

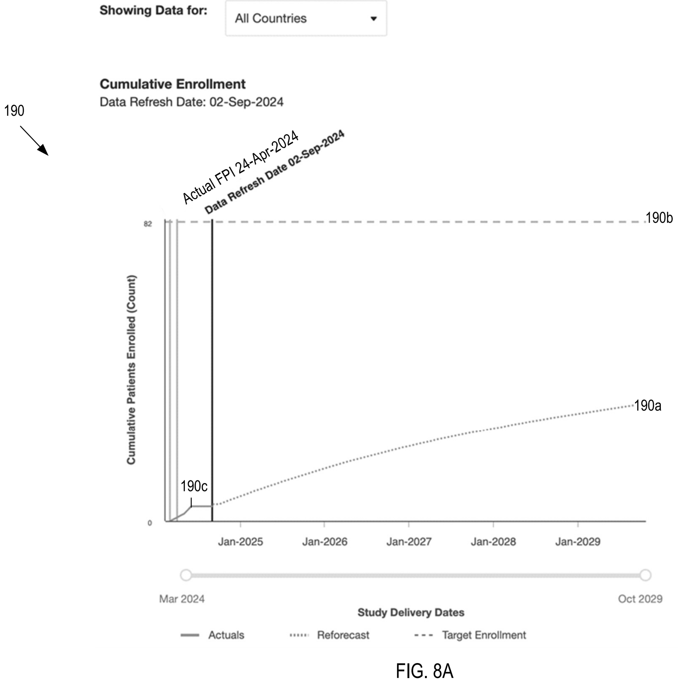

Referring to FIG. 8A, graphical user interface 190 is shown of an aggregated study enrollment forecast. Graphical user interface 190 includes visual representation 190a that specifies a predicted enrollment by various dates, visual representation 190b that specifies a target enrollment and visual representation 190c that specifies the actual enrollment.

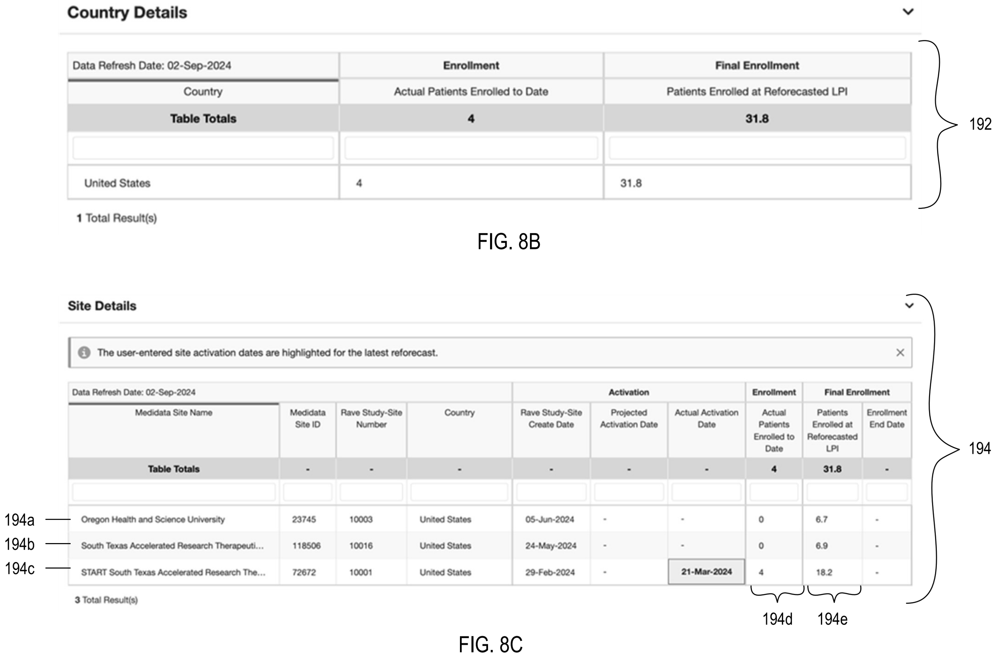

Referring to FIG. 8B, graphical user interface 192 is shown that specifies an aggregated view of predicted enrollment for a particular study. In this example, the aggregated view is at the level of a country.

Referring to FIG. 8C, graphical user interface 194 is shown that provides a more granular view of the predictions for the various sites. In this example, graphical user interface 194 includes rows 194a-194c, each specifying a particular site. Graphical user interface 194 also includes column 194d that specifies actual enrollments to-date for the sites and column 194e which specifies predictions for the sites.

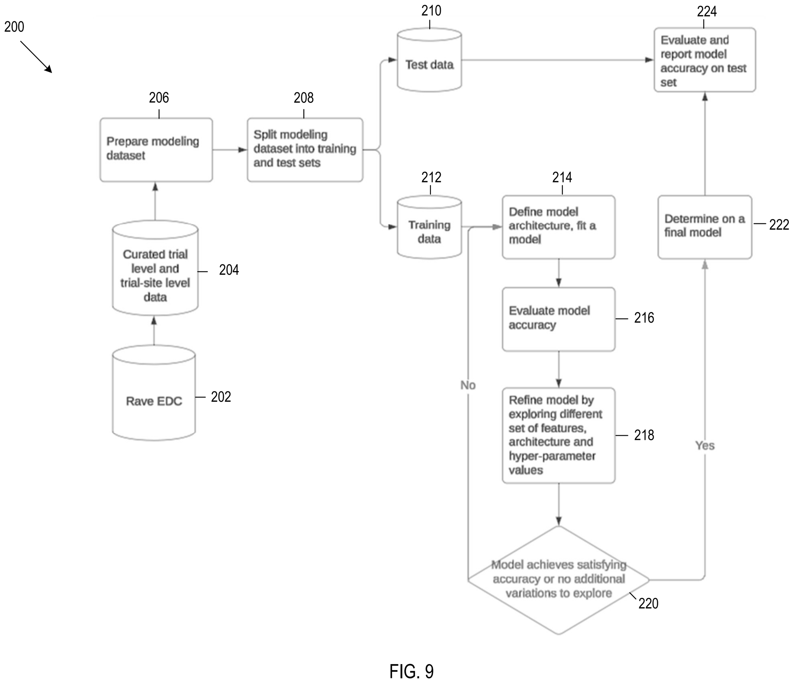

Referring to FIG. 9, process 200 is shown to develop and validate the neural network to perform the enrollment predictions as described herein. In this example, EDC system 202 accesses trial level data 204 and trial-site level data. The data processing system described herein uses the trial level data 204 to prepare a modeling dataset 206. Preparation of the modeling dataset is described herein. The data processing system splits 208 the modeling dataset into training and test sets as described herein. The training data is stored in data store 212. Test data is stored in data store 210. The data processing system defines 214 a model architecture and fits a model. The data processing system evaluates 216 the model accuracy. The data processing system refines 218 the model by exploring different sets of features, architecture, and hyper parameter values. The data processing system determines 220 whether the model achieves satisfying accuracy, or whether there are no additional variations to explore. When the model does not achieve satisfying accuracy, or there are additional variations to explore, then the data processing system returns to defining and fitting the model. However, when the data processing system determines that the model achieves satisfying accuracy, or that there are no additional variations to explore, the data processing system determines 222 a final model. The data processing system then uses the test data that is stored in data store 210 to test that final model and evaluate and report 224 model accuracy on the test data.

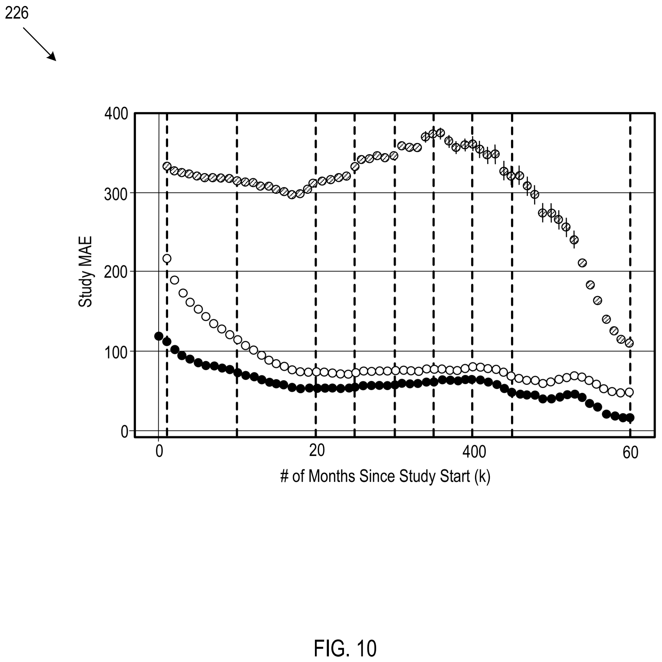

Referring to FIG. 10, visualization 226 of model (e.g., the neural network) performance on a test set with over 2,000 historical studies is shown. For each study, the aggregated predicted enrollment count at the study last-patient-in date is compared with the study's actual enrollment count and the absolute error (AE) is calculated. This exercise is conducted assuming different months of known enrollment (k), from 1 to 60. The AEs calculated at the same k are averaged across studies, arriving at the mean absolute error (MAE). Each MAE value corresponds to one dot in the figure below, and the lower values represent the better model performance.

Additionally, model performance is compared to several baselines with several baselines. The solid black line (e.g., the lowest line) is the deep learning model's performance, the hatched line (e.g., the top line) is the baseline that predicts future enrollment using within-country historical enrollment rate, and the middle line (the line with white circles) is one that predicts future enrollment rate by using the average observed enrollment rate of the current study up-to month k. The deep learning model (e.g., the neural network described herein) outperforms both baseline methods.

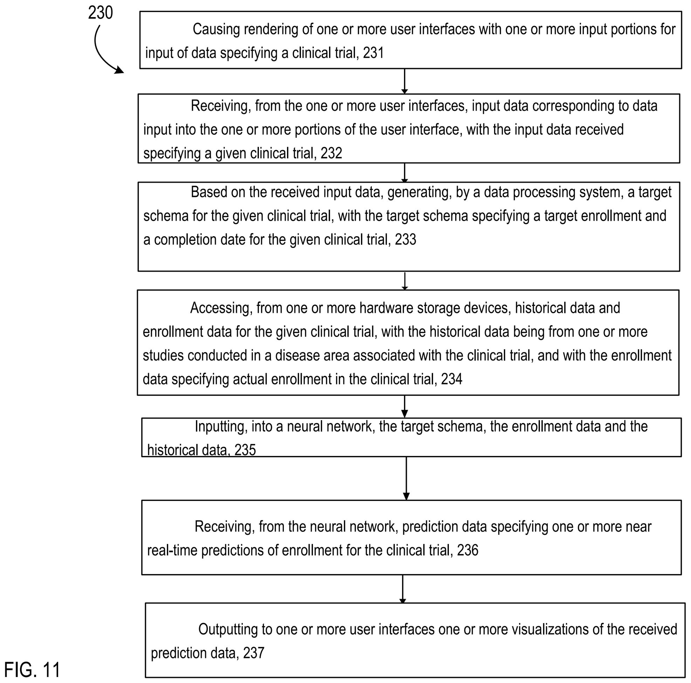

Referring to FIG. 11, process 230 is shown for generating a target schema for use with a neural network to generate output indicative of a near real-time prediction of clinical study enrollment. In operation, a data processing system causes (231) rendering of one or more user interfaces with one or more input portions for input of data specifying a clinical trial. The data processing system receives (232), input data corresponding to data input into the one or more portions of the user interface, with the input data received specifying a given clinical trial. Based on the received input data, the data processing system generates (233) a target schema for the given clinical trial, with the target schema specifying a target enrollment and a completion date for the given clinical trial. In some examples, the target enrollment and the completion data are input into the graphical user interfaces. In other examples, the data processing system uses the input of data specifying the clinical trial (e.g., a name of the clinical trial) to look-up or retrieve from a data store information about the clinical trial, e.g., target enrollment, start date, completion date, site names, etc.).

The data processing system accesses (234), from one or more hardware storage devices, historical data and enrollment data for the given clinical trial, with the historical data being from one or more studies conducted in a disease area associated with the clinical trial, and with the enrollment data specifying actual enrollment in the clinical trial. In this example, the data processing system requests from CTMS a disease area of the clinical trial study for which the prediction is generated and then requests historical data (e.g., historical enrollment data) for clinical trial studies in a same or similar disease area. The data processing system also retrieves from CTMS actual enrollment data for the clinical trial study for which the prediction is generated.

The data processing system inputs (235), into a neural network, the target schema, the enrollment data and the historical data. The data processing system receives (236), from the neural network, prediction data specifying one or more near real-time predictions of enrollment for the clinical trial. The data processing system outputs (237) one or more user interfaces one or more visualizations of the received prediction data.

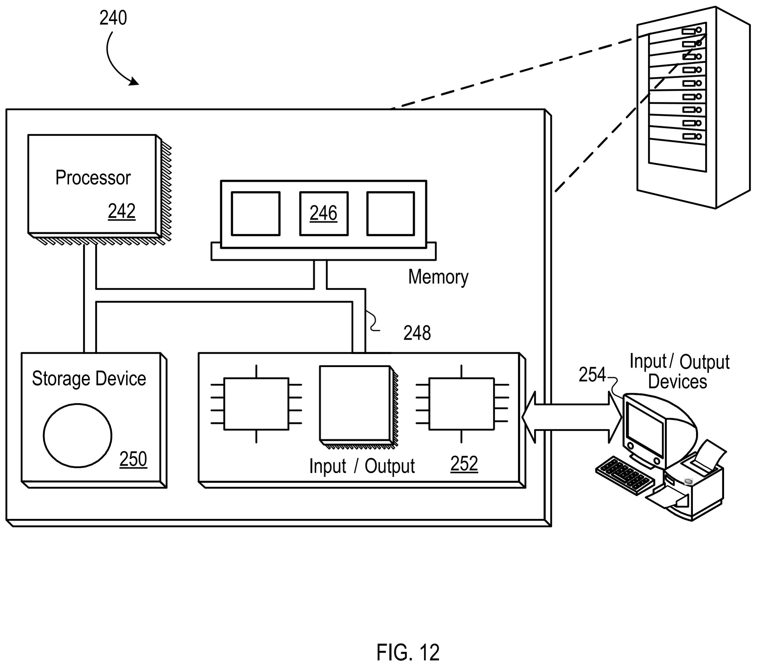

FIG. 12 depicts an example computing system, according to implementations of the present disclosure. The system 240 may be used for any of the operations described with respect to the various implementations discussed herein. The system 240 may include one or more processors 242, a memory 246, one or more storage devices 250, and one or more input/output (I/O) devices 254 controllable through one or more I/O interfaces 252. The various components 242, 246, 250, 252, or 254 may be interconnected through at least one system bus 248, which may enable the transfer of data between the various modules and components of the system 240.

The processor(s) 242 may be configured to process instructions for execution within the system 240. The processor(s) 242 may include single-threaded processor(s), multi-threaded processor(s), or both. The processor(s) 242 may be configured to process instructions stored in the memory 246 or on the storage device(s) 250. The processor(s) 242 may include hardware-based processor(s), each including one or more cores. The processor(s) 242 may include general purpose processor(s), special purpose processor(s), or both.

The memory 246 may store information within the system 240. In some implementations, the memory 246 includes one or more computer-readable media. The memory 246 may include any number of volatile memory units, any number of non-volatile memory units, or both volatile and non-volatile memory units. The memory 246 may include read-only memory, random access memory, or both. In some examples, the memory 246 may be employed as active or physical memory by one or more executing software modules.

The storage device(s) 250 may be configured to provide (e.g., persistent) mass storage for the system 240. In some implementations, the storage device(s) 250 may include one or more computer-readable media. For example, the storage device(s) 250 may include a floppy disk device, a hard disk device, an optical disk device, or a tape device. The storage device(s) 250 may include read-only memory, random access memory, or both. The storage device(s) 250 may include one or more of an internal hard drive, an external hard drive, or a removable drive.

One or both of the memory 246 or the storage device(s) 250 may include one or more computer-readable storage media (CRSM), including, e.g., a non-transitory computer readable medium. The CRSM may include one or more of an electronic storage medium, a magnetic storage medium, an optical storage medium, a magneto-optical storage medium, a quantum storage medium, a mechanical computer storage medium, and so forth. The CRSM may provide storage of computer-readable instructions describing data structures, processes, applications, programs, other modules, or other data for the operation of the system 240. In some implementations, the CRSM may include a data store that provides storage of computer-readable instructions or other information in a non-transitory format. The CRSM may be incorporated into the system 240 or may be external with respect to the system 240. The CRSM may include read-only memory, random access memory, or both. One or more CRSM suitable for tangibly embodying computer program instructions and data may include any type of non-volatile memory, including but not limited to: semiconductor memory devices, such as EPROM, EEPROM, and flash memory devices; magnetic disks such as internal hard disks and removable disks; magneto-optical disks; and CD-ROM and DVD-ROM disks. In some examples, the processor(s) 242 and the memory 246 may be supplemented by, or incorporated into, one or more application-specific integrated circuits (ASICs).

The system 240 may include one or more I/O devices 254. The I/O device(s) 254 may include one or more input devices such as a keyboard, a mouse, a pen, a game controller, a touch input device, an audio input device (e.g., a microphone), a gestural input device, a haptic input device, an image or video capture device (e.g., a camera), or other devices. In some examples, the I/O device(s) 254 may also include one or more output devices such as a display, LED(s), an audio output device (e.g., a speaker), a printer, a haptic output device, and so forth. The I/O device(s) 254 may be physically incorporated in one or more computing devices of the system 240, or may be external with respect to one or more computing devices of the system 240.

The system 240 may include one or more I/O interfaces 252 to enable components or modules of the system 240 to control, interface with, or otherwise communicate with the I/O device(s) 254. The I/O interface(s) 252 may enable information to be transferred in or out of the system 240, or between components of the system 240, through serial communication, parallel communication, or other types of communication. For example, the I/O interface(s) 252 may comply with a version of the RS-232 standard for serial ports, or with a version of the IEEE 1284 standard for parallel ports. As another example, the I/O interface(s) 252 may be configured to provide a connection over Universal Serial Bus (USB) or Ethernet. In some examples, the I/O interface(s) 252 may be configured to provide a serial connection that is compliant with a version of the IEEE 1394 standard.

The I/O interface(s) 252 may also include one or more network interfaces that enable communications between computing devices in the system 240, or between the system 240 and other network-connected computing systems. The network interface(s) may include one or more network interface controllers (NICs) or other types of transceiver devices configured to send and receive communications over one or more networks using any network protocol.

Computing devices of the system 240 may communicate with one another, or with other computing devices, using one or more networks. Such networks may include public networks such as the internet, private networks such as an institutional or personal intranet, or any combination of private and public networks. The networks may include any type of wired or wireless network, including but not limited to local area networks (LANs), wide area networks (WANs), wireless WANs (WWANs), wireless LANs (WLANs), mobile communications networks (e.g., 3G, 4G, Edge, etc.), and so forth. In some implementations, the communications between computing devices may be encrypted or otherwise secured. For example, communications may employ one or more public or private cryptographic keys, ciphers, digital certificates, or other credentials supported by a security protocol, such as any version of the Secure Sockets Layer (SSL) or the Transport Layer Security (TLS) protocol.

The system 240 may include any number of computing devices of any type. The computing device(s) may include, but are not limited to: a personal computer, a smartphone, a tablet computer, a wearable computer, an implanted computer, a mobile gaming device, an electronic book reader, an automotive computer, a desktop computer, a laptop computer, a notebook computer, a game console, a home entertainment device, a network computer, a server computer, a mainframe computer, a distributed computing device (e.g., a cloud computing device), a microcomputer, a system on a chip (SoC), a system in a package (SiP), and so forth. Although examples herein may describe computing device(s) as physical device(s), implementations are not so limited. In some examples, a computing device may include one or more of a virtual computing environment, a hypervisor, an emulation, or a virtual machine executing on one or more physical computing devices. In some examples, two or more computing devices may include a cluster, cloud, farm, or other grouping of multiple devices that coordinate operations to provide load balancing, failover support, parallel processing capabilities, shared storage resources, shared networking capabilities, or other aspects.

This specification uses the term "configured" in connection with systems and computer program components. For a system of one or more computers to be configured to perform particular operations or actions means that the system has installed on its software, firmware, hardware, or a combination of them that in operation cause the system to perform the operations or actions. For one or more computer programs to be configured to perform particular operations or actions means that the one or more programs include instructions that, when executed by data processing apparatus, cause the apparatus to perform the operations or actions.

Embodiments of the subject matter and the functional operations described in this specification can be implemented in digital electronic circuitry, in tangibly-embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non transitory storage medium for execution by, or to control the operation of, data processing apparatus. The computer storage medium can be a machine-readable storage device, a machine-readable storage substrate, a random or serial access memory device, or a combination of one or more of them. Alternatively, or in addition, the program instructions can be encoded on an artificially generated propagated signal, e.g., a machine-generated electrical, optical, or electromagnetic signal, that is generated to encode information for transmission to suitable receiver apparatus for execution by a data processing apparatus.

The term data processing apparatus (also referred to herein as a data processing system) refers to data processing hardware and encompasses all kinds of apparatus, devices, and machines for processing data, including by way of example a programmable processor, a computer, or multiple processors or computers. The apparatus can also be, or further include, special purpose logic circuitry, e.g., an FPGA (field programmable gate array) or an ASIC (application specific integrated circuit). The apparatus can optionally include, in addition to hardware, code that creates an execution environment for computer programs, e.g., code that constitutes processor firmware, a protocol stack, a database management system, an operating system, or a combination of one or more of them.

A computer program, which may also be referred to or described as a program, software, a software application, an app, a module, a software module, a script, or code, can be written in any form of programming language, including compiled or interpreted languages, or declarative or procedural languages; and it can be deployed in any form, including as a standalone program or as a module, component, subroutine, or other unit suitable for use in a computing environment. A program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub programs, or portions of code. A computer program can be deployed to be executed on one computer or on multiple computers that are located at one site or distributed across multiple sites and interconnected by a data communication network.

In this specification, the term database is used broadly to refer to any collection of data: the data does not need to be structured in any particular way, or structured at all, and it can be stored on storage devices in one or more locations. Thus, for example, the index database can include multiple collections of data, each of which may be organized and accessed differently.

Similarly, in this specification the term engine is used broadly to refer to a software-based system, subsystem, or process that is programmed to perform one or more specific functions. Generally, an engine will be implemented as one or more software modules or components, installed on one or more computers in one or more locations. In some cases, one or more computers will be dedicated to a particular engine; in other cases, multiple engines can be installed and running on the same computer or computers.

The processes and logic flows described in this specification can be performed by one or more programmable computers executing one or more computer programs to perform functions by operating on input data and generating output. The processes and logic flows can also be performed by special purpose logic circuitry, e.g., an FPGA or an ASIC, or by a combination of special purpose logic circuitry and one or more programmed computers.

Computers suitable for the execution of a computer program can be based on general or special purpose microprocessors or both, or any other kind of central processing unit. Generally, a central processing unit will receive instructions and data from a read only memory or a random access memory or both. The essential elements of a computer are a central processing unit for performing or executing instructions and one or more memory devices for storing instructions and data. The central processing unit and the memory can be supplemented by, or incorporated in, special purpose logic circuitry. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

Computer readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD-ROM disks.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's device in response to requests received from the web browser. Also, a computer can interact with a user by sending text messages or other forms of messages to a personal device, e.g., a smartphone that is running a messaging application, and receiving responsive messages from the user in return.

Embodiments of the subject matter described in this specification can be implemented in a computing system that includes a back end component, e.g., as a data server, or that includes a middleware component, e.g., an application server, or that includes a front end component, e.g., a client computer having a graphical user interface, a web browser, or an app through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network (LAN) and a wide area network (WAN), e.g., the Internet.

The computing system can include clients and servers. A client and server are generally remote from each other and typically interact through a communication network. The relationship of client and server arises by virtue of computer programs running on the respective computers and having a client-server relationship to each other. In some embodiments, a server transmits data, e.g., an HTML page, to a user device, e.g., for purposes of displaying data to and receiving user input from a user interacting with the device, which acts as a client. Data generated at the user device, e.g., a result of the user interaction, can be received at the server from the device.

While this specification contains many specific implementation details, these should not be construed as limitations on the scope of any invention or on the scope of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially be claimed as such, one or more features from a claimed combination can in some cases be excised from the combination, and the claimed combination may be directed to a sub-combination or variation of a sub-combination.

Similarly, while operations are depicted in the drawings and recited in the claims in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described above should not be understood as requiring such separation in all embodiments, and it should be understood that the described program components and systems can generally be integrated together in a single software product or packaged into multiple software products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In some cases, multitasking and parallel processing may be advantageous.

### EMBODIMENTS

Although the present invention is defined in the claims, it should be understood that the present invention can also (alternatively) be defined in accordance with the following embodiments:
1. An apparatus for facilitating development of software to use a neural network in generating output indicative of a near real-time prediction of clinical study enrollment, comprising:
   one or more processors; and
   one or more hardware storage devices storing instructions that are executable by the one or more processors to perform one or more first operations comprising:
      receiving, in a development environment, one or more instructions that are executable by the one or more processors to perform one or more second operations comprising:
      causing rendering of one or more user interfaces with one or more input portions for input of data specifying a clinical trial;
      receiving, from the one or more user interfaces, input data corresponding to data input into the one or more portions of the user interface, with the input data received specifying a given clinical trial;
      receiving, from the one or more user interfaces, a target schema for the given clinical trial;
      accessing, from one or more hardware storage devices, historical data and enrollment data for the given clinical trial, with the historical data being from one or more studies conducted in a disease area associated with the clinical trial, and with the enrollment data specifying actual enrollment in the clinical trial;
      inputting, into a neural network, the target schema, received input data specifying the given clinical trial, the enrollment data and the historical data;
      receiving, from the neural network, prediction data specifying one or more near real-time predictions of enrollment for the clinical trial; and
      outputting to one or more user interfaces one or more visualizations of the received prediction data.
2. The apparatus of embodiment 1, wherein the clinical trial is associated with one or more sites, wherein the accessed enrollment data is for a given one of the one or more sites, and wherein the prediction data specifies one or more near real-time predictions of enrollment for the clinical trial for the given one of the one or more sites.
3. The apparatus of embodiment 1, wherein the target schema specifies an enrollment target, an activation date and/or a completion date.
4. The apparatus of embodiment 1, wherein the neural network is a recurrent neural network.
5. The apparatus of embodiment 1, wherein the one or more visualizations provide a first view at a study level or a second view at a country level.
6. A method implemented by a data processing system for generating a target schema for use with a neural network to generate output indicative of a near real-time prediction of clinical study enrollment, comprising:
   causing rendering of one or more user interfaces with one or more input portions for input of data specifying a clinical trial;
   receiving, from the one or more user interfaces, input data corresponding to data input into the one or more portions of the user interface, with the input data received specifying a given clinical trial;
   based on the received input data, generating, by a data processing system, a target schema for the given clinical trial, with the target schema specifying a target enrollment and a completion date for the given clinical trial;
   accessing, from one or more hardware storage devices, historical data and enrollment data for the given clinical trial, with the historical data being from one or more studies conducted in a disease area associated with the clinical trial, and with the enrollment data specifying actual enrollment in the clinical trial;
   inputting, into a neural network, the target schema, the enrollment data and the historical data;
   receiving, from the neural network, prediction data specifying one or more near real-time predictions of enrollment for the clinical trial; and
   outputting to one or more user interfaces one or more visualizations of the received prediction data.
7. The method of embodiment 6, wherein the clinical trial is associated with one or more sites, wherein the accessed enrollment data is for a given one of the one or more sites, and wherein the prediction data specifies one or more near real-time predictions of enrollment for the clinical trial for the given one of the one or more sites.
8. The method of embodiment 6, wherein the target schema specifies an enrollment target, an activation date and/or a completion date.
9. The method of embodiment 6, further comprising:
   calibrating the neural network by:
   accessing, from a hardware storage device, clinical trial data;
   segmenting the clinical trial data into test data and training data;
   for the training data,
      identifying one or more features of the training data;
      encoding the one or more features;
      transmitting at least a portion of the one or more encoded features to one or more hidden layers of the neural network;
      based on an output of the one or more hidden layers, determining one or more values of one or more hyperparameters of the neural network; and
      repeating the transmitting and the determining until the neural network outputs one or more values satisfying one or more thresholds.
10. The method of embodiment 6, further comprising:
   training the neural network to predict the one or more near real-time predictions using a supervised learning technique based on training data, wherein the neural network is configured to receive as input the target schema, input data specifying the given clinical trial, the enrollment data and the historical data and to process the input to generate an output that specifies one or more near real-time predictions of enrollment for the given clinical trial, wherein the neural network comprises a plurality of artificial neurons that are connected through edges and are aggregated into a plurality of neural network layers comprising at least an input layer and an output layer, wherein each of the edges is configured to transmit a signal from one artificial neuron to another artificial neuron, and wherein an output of each of the plurality of artificial neurons is computed based on inputs of the artificial neuron in accordance with a plurality of weights.
11. The method of embodiment 10, further comprising:
   processing, by the data processing system, new data using the plurality of artificial neurons in the trained neural network in accordance with the values of the plurality of weights to identify the one or more near real-time predictions of enrollment for the given clinical trial, wherein the artificial neurons in the input layer are configured to receive the new data as input and the artificial neurons in the output layer are configured to generate a new output that identifies a near real-time prediction of enrollment, and wherein the new data includes the target schema and the received input data.
12. The method of embodiment 6, further comprising:
   training the neural network to predict enrollment, comprising:
   receiving training data, with one or more portions of the training data being associated with a label specifying an enrollment count;
   defining a first squared error function that quantifies a prediction error of the neural network based on the training data; and
   setting weights of the neural network to minimize the first prediction error.
13. The method of embodiment 6, wherein the neural network is a recurrent neural network.
14. The method of embodiment 6, wherein the one or more visualizations provide a first view at a study level or a second view at a country level.
15. The method of embodiment 6, further including:
   determining that at least one or more near real-time predictions specifies enrollment that is less than a threshold amount;
   responsive to the determining, generating functional data that when received by a clinical trial management system causes the clinical trial management system to perform one or more operations, wherein at least one of the one or more operations is allocating memory and processing resources for generating one or more communications and transmitting over a network the one or more communications to one or more client devices, wherein each client device is associated with a site of the clinical trial study and a communication informs a user of a client device that has received the communication that the enrollment is less than a threshold amount and prompts the user to perform one or more actions; and
   transmitting the functional data to a clinical trial management system.
16. The method of embodiment 15, wherein the functional data is an alert.
17. One or more machine-readable hardware storage devices for generating a target schema for use with a neural network to generate output indicative of a near real-time prediction of clinical study enrollment, the one or more machine-readable hardware storage devices storing instructions that are executable by one or more processors to perform operations comprising:
   causing rendering of one or more user interfaces with one or more input portions for input of data specifying a clinical trial;
   receiving, from the one or more user interfaces, input data corresponding to data input into the one or more portions of the user interface, with the input data received specifying a given clinical trial;
   based on the received input data, generating, by a data processing system, a target schema for the given clinical trial, with the target schema specifying a target enrollment and a completion date for the given clinical trial;
   accessing, from one or more hardware storage devices, historical data and enrollment data for the given clinical trial, with the historical data being from one or more studies conducted in a disease area associated with the clinical trial, and with the enrollment data specifying actual enrollment in the clinical trial;
   inputting, into a neural network, the target schema, the enrollment data and the historical data;
   receiving, from the neural network, prediction data specifying one or more near real-time predictions of enrollment for the clinical trial; and
   outputting to one or more user interfaces one or more visualizations of the received prediction data.
18. The one or more machine-readable hardware storage devices of embodiment 17, wherein the clinical trial is associated with one or more sites, wherein the accessed enrollment data is for a given one of the one or more sites, and wherein the prediction data specifies one or more near real-time predictions of enrollment for the clinical trial for the given one of the one or more sites.
19. The one or more machine-readable hardware storage devices of embodiment 17, wherein the target schema specifies an enrollment target, an activation date and/or a completion date.
20. The one or more machine-readable hardware storage devices of embodiment 17, wherein the operations further comprise:
   calibrating the neural network by:
   accessing, from a hardware storage device, clinical trial data;
   segmenting the clinical trial data into test data and training data;
   for the training data,
      identifying one or more features of the training data;
      encoding the one or more features;
      transmitting at least a portion of the one or more encoded features to one or more hidden layers of the neural network;
      based on an output of the one or more hidden layers, determining one or more values of one or more hyperparameters of the neural network; and
      repeating the transmitting and the determining until the neural network outputs one or more values satisfying one or more thresholds.

## Claims

1. An apparatus for facilitating development of software to use a neural network in generating output indicative of a near real-time prediction of clinical study enrollment, comprising:
one or more processors; and
one or more hardware storage devices storing instructions that are executable by the one or more processors to perform one or more first operations comprising:
receiving, in a development environment, one or more instructions that are executable by the one or more processors to perform one or more second operations comprising:
causing rendering of one or more user interfaces with one or more input portions for input of data specifying a clinical trial;
receiving, from the one or more user interfaces, input data corresponding to data input into the one or more portions of the user interface, with the input data received specifying a given clinical trial;
receiving, from the one or more user interfaces, a target schema for the given clinical trial;
accessing, from one or more hardware storage devices, historical data and enrollment data for the given clinical trial, with the historical data being from one or more studies conducted in a disease area associated with the clinical trial, and with the enrollment data specifying actual enrollment in the clinical trial;
inputting, into a neural network, the target schema, received input data specifying the given clinical trial, the enrollment data and the historical data;
receiving, from the neural network, prediction data specifying one or more near real-time predictions of enrollment for the clinical trial; and
outputting to one or more user interfaces one or more visualizations of the received prediction data.

2. The apparatus of claim 1, wherein the clinical trial is associated with one or more sites, wherein the accessed enrollment data is for a given one of the one or more sites, and wherein the prediction data specifies one or more near real-time predictions of enrollment for the clinical trial for the given one of the one or more sites.

3. The apparatus of any one of claims 1 or 2, wherein the target schema specifies an enrollment target, an activation date and/or a completion date.

4. The apparatus of any one of claims 1 to 3, wherein the neural network is a recurrent neural network; and/or
wherein the one or more visualizations provide a first view at a study level or a second view at a country level.

5. A method implemented by a data processing system for generating a target schema for use with a neural network to generate output indicative of a near real-time prediction of clinical study enrollment, comprising:
causing rendering of one or more user interfaces with one or more input portions for input of data specifying a clinical trial;
receiving, from the one or more user interfaces, input data corresponding to data input into the one or more portions of the user interface, with the input data received specifying a given clinical trial;
based on the received input data, generating, by a data processing system, a target schema for the given clinical trial, with the target schema specifying a target enrollment and a completion date for the given clinical trial;
accessing, from one or more hardware storage devices, historical data and enrollment data for the given clinical trial, with the historical data being from one or more studies conducted in a disease area associated with the clinical trial, and with the enrollment data specifying actual enrollment in the clinical trial;
inputting, into a neural network, the target schema, the enrollment data and the historical data;
receiving, from the neural network, prediction data specifying one or more near real-time predictions of enrollment for the clinical trial; and
outputting to one or more user interfaces one or more visualizations of the received prediction data.

6. The method of claim 5, wherein the clinical trial is associated with one or more sites, wherein the accessed enrollment data is for a given one of the one or more sites, and wherein the prediction data specifies one or more near real-time predictions of enrollment for the clinical trial for the given one of the one or more sites; and/or
wherein the target schema specifies an enrollment target, an activation date and/or a completion date.

7. The method of any one of claims 5 or 6, further comprising:
calibrating the neural network by:
accessing, from a hardware storage device, clinical trial data;
segmenting the clinical trial data into test data and training data;
for the training data,
identifying one or more features of the training data;
encoding the one or more features;
transmitting at least a portion of the one or more encoded features to one or more hidden layers of the neural network;
based on an output of the one or more hidden layers, determining one or more values of one or more hyperparameters of the neural network; and
repeating the transmitting and the determining until the neural network outputs one or more values satisfying one or more thresholds.

8. The method of any one of claims 6 or 7, further comprising:
training the neural network to predict the one or more near real-time predictions using a supervised learning technique based on training data, wherein the neural network is configured to receive as input the target schema, input data specifying the given clinical trial, the enrollment data and the historical data and to process the input to generate an output that specifies one or more near real-time predictions of enrollment for the given clinical trial, wherein the neural network comprises a plurality of artificial neurons that are connected through edges and are aggregated into a plurality of neural network layers comprising at least an input layer and an output layer, wherein each of the edges is configured to transmit a signal from one artificial neuron to another artificial neuron, and wherein an output of each of the plurality of artificial neurons is computed based on inputs of the artificial neuron in accordance with a plurality of weights.

9. The method of claim 8, further comprising:
processing, by the data processing system, new data using the plurality of artificial neurons in the trained neural network in accordance with the values of the plurality of weights to identify the one or more near real-time predictions of enrollment for the given clinical trial, wherein the artificial neurons in the input layer are configured to receive the new data as input and the artificial neurons in the output layer are configured to generate a new output that identifies a near real-time prediction of enrollment, and wherein the new data includes the target schema and the received input data.

10. The method of any one of claims 5 to 9, further comprising:
training the neural network to predict enrollment, comprising:
receiving training data, with one or more portions of the training data being associated with a label specifying an enrollment count;
defining a first squared error function that quantifies a prediction error of the neural network based on the training data; and
setting weights of the neural network to minimize the first prediction error.

11. The method of any one of claims 5 to 10, wherein the neural network is a recurrent neural network.

12. The method of any one of claims 5 to 11, wherein the one or more visualizations provide a first view at a study level or a second view at a country level.

13. The method of any one of claims 5 to 12, further including:
determining that at least one or more near real-time predictions specifies enrollment that is less than a threshold amount;
responsive to the determining, generating functional data that when received by a clinical trial management system causes the clinical trial management system to perform one or more operations, wherein at least one of the one or more operations is allocating memory and processing resources for generating one or more communications and transmitting over a network the one or more communications to one or more client devices, wherein each client device is associated with a site of the clinical trial study and a communication informs a user of a client device that has received the communication that the enrollment is less than a threshold amount and prompts the user to perform one or more actions; and
transmitting the functional data to a clinical trial management system.

14. The method of claim 13, wherein the functional data is an alert.

15. One or more machine-readable hardware storage devices for generating a target schema for use with a neural network to generate output indicative of a near real-time prediction of clinical study enrollment, the one or more machine-readable hardware storage devices storing instructions that are executable by one or more processors to perform the method of any one of claims 5 to 14.
